# EUROPEAN PATENT SPECIFICATION

(11) **EP 3 151 752 B1**
(45) Date of publication and mention of the grant of the patent: **16.05.2018**
(21) Application number: 15729113.9
(22) Date of filing: 03.06.2015
(51) Int. Cl.: A61B 6/10, A61B 90/00

(54) **THYROID PROTECTOR**
SCHILDDRÜSENSCHUTZVORRICHTUNG
PROTECTEUR DE LA THYROÏDE

(30) Priority: 03.06.2014 NL 2012936
(43) Date of publication of application: 12.04.2017
(73) Proprietor: Gentleceph B.V., 3062 KK Rotterdam (NL)
(72) Inventor: HOOGEVEEN, Reinier Cornelis, 3062 KK Rotterdam (NL)
(74) Representative: EP&C
(86) International application number: PCT/EP2015/062368
(87) International publication number: WO 2015/185617

(56) References cited:
- US-A- 3 569 713
- US-A- 4 024 405
- US-A- 5 778 888
- US-A1- 2006 243 286
- US-A1- 2010 186 753
- US-A1- 2012 010 517

## Description

The invention relates to a radiation protector for protecting a thyroid of a person from x-ray radiation, an x-ray device, and a method for taking an x-ray image.

US3.569.713 is considered to be the closest prior art and discloses a thyroid gland protector including a shield pad made of a material which is substantially impervious to x-rays. The shield pad has a crescent shape. The shield pad has a height at a centre region which extends after placement to a neck of a person from the chin to the sternum. The shield pad is configured to encompass the forward area of the neck as a collar to shield the larynx and the thyroid from x-ray radiation. The ends of the shield pad extend beyond the back of the neck so that the ends can contact each other. A clip of the spring pressed pincer type is used to secure the two ends together.

The known protector is provided for a universal use for all people. The known protector is arranged to be used for both adults and children. When the protector is used for children, it appears to be difficult to place the protector correctly. Frequently, the protector fits poorly in between the chin and the sternum. The uncomfortable protector generates an unpleasant experience to a person from who an x-ray image needs to be taken. Additionally, this unpleasant experience might contribute to an already present fear of a person to be treated.

Since the protector is to be used for several persons and directly placed to a skin of a person, the protector needs to be cleaned after each use. In use, the protector becomes contaminated by hairs and sebum. US2010/0186753 discloses a thyroid shield cover to be used to protect a thyroid shield from becoming soiled with blood and other bodily fluids during radiological procedures. The thyroid shield cover is made of a surgical paper. The thyroid shield cover has an elongated shape and has a folding line in its length direction. An operator has to fold the thyroid shield cover over and around the thyroid shield before placing the protector around a neck of a person. The cleaning of the known protector by replacing the cover requires some manual operations which has to be carried out frequently and accurately. In practise, the placement of the cover appears to be clumsy. In particular, in a busy dental practice, it is undesired to spend much time and attention for cleaning a protector. In particular, the folding step is undesirable, because this requires some special attention of a medical specialist to fold exactly along the folding line. The additional step of placing the shield inside the folded cover provides a further problem in that the shield might shift during the operational steps.

Because of these problems of a inconvenient placement and cumbersome cleaning, in a worst case, the placement of the radiation protector might be omitted which may lead to an undesired exposure to x-ray radiation.

The general object of the present invention is to at least partially eliminate the above mentioned drawbacks and/or to provide a useable alternative.

More specific, it is an object of the invention to provide a radiation protector which is comfortable in use. It is an object of the invention to provide a radiation protector which allows a correct placement in a simple manner. Further, it is an object of the invention to provide a radiation protector which can be cleaned easily.

According to the invention, this object is achieved by a radiation protector according to claim 1.

According to the invention a radiation protector is provided for protecting a thyroid of a person from x-ray radiation. The radiation protector comprises a shield pad shielding the thyroid which is a body part to be protected. The shield pad comprises a layer of shield material for attenuating the flux of electromagnetic radiation. The shield pad may attenuate a flux to comply to legal regulations on x-ray radiation. In particular, the shield pad may attenuate a flux to at most 50%, in particular at most 30%. Preferably, the shield pad is substantially impervious to x-ray radiation. Further, the radiation protector comprises a fastener for fastening the shield pad to the thyroid. The shield pad is connectable by the fastener in abutting engagement with the thyroid to be protected.

The radiation protector according to the invention is improved in that the fastener comprises a resilient U-shaped bracket. The bracket has a first end at a first U-leg. The first end is connected to the shield pad. In use, the shield pad has to be placed at the thyroid to be protected. The bracket has a second end at a second U-leg. The second end is situated opposite the first end, such that in use the second end engages an area of a person's body situated opposed the thyroid to be protected, e.g. at the occiput or nape. The thyroid to be protected is situated in between the two ends, in particular in between the first and second U-leg, of the bracket when the radiation protector is clamped to the thyroid.

Advantageously, the U-shaped bracket of the radiation protector according to the invention enhances a quick and correct placement of the radiation protector to the thyroid to be protected. The bracket is resilient. The U-shaped bracket exerts a clamping force for clamping the thyroid to be protected in between the first and second leg to maintain the shield pad at the thyroid. Particularly, the U-shaped bracket is advantageous in the use of the radiation protector for children. Instead of closing a shield pad all around a neck of a child, the U-shaped bracket allows a placement of the radiation protector from aside which is more convenient to the child.

In an embodiment of the radiation protector according to the invention, the bracket is pivotally connected to the shield pad. The bracket is connected by a pivotal joint to the shield pad. The pivotal joint allows a rotational movement of the shield pad with respect to the bracket. Advantageously, the bracket can be moved away from an area to be imaged, such that the bracket does less disturb an imaging process.

In an embodiment of the radiation protector according to the invention, the shield pad is relatively small in comparison with a collar shaped shield pad. Preferably, the shield pad has a minimal pad area which suffices to cover predetermined sensitive organs. The shield pad according to the invention may include a pad area of at most 50 cm², in particular at most 40 cm², but preferably at most 30 cm². Preferably, the small shield pad is only connected to the first end of the bracket. Preferably, the shield pad has an anatomic shape.

In an alternative embodiment of the radiation protector according to the invention, the shield pad may be elongated and connected to both the first and second end of the bracket. The shield pad extends in between the first and second leg of the U-shaped bracket. Particularly, the U-shaped bracket extends in parallel with the shield pad. The U-shaped bracket is arranged to clamp the shield pad around a body part of a person. In use as a thyroid protector, the elongated shield pad may circumvent a left or right side of a neck of a person.

In an embodiment of the radiation protector according to the invention, the bracket has a free second end at the second U-leg. In use, the second end of the bracket may directly contact a skin of a person. The bracket may be made from a metal or preferably plastic material which is in use at the second end in direct contact with the person. Preferably, the second end of the bracket is provided with a clamp pad for cushioning an engagement of the second end. The clamp pad is separate from the shield pad. The clamp pad provides advantageously a more comfortable contact with a skin of a person.

In an embodiment of the radiation protector according to the invention, the bracket is made from a plastic material. Advantageously, in comparison with a metal material, the plastic material does less affect x-ray imaging.

In an embodiment of the radiation protector according to the invention, the protector comprises a removable cover for covering the shield pad and/or the clamp pad. The removable cover is attachable to the shield pad and/or the clamp pad. Preferably, the cover is bag shaped. The bag shaped cover has an opening which fits around the shield pad. Preferably, the opening is arranged elastically or closable by a closure, like a chord, to fasten the cover to the shield pad. The removable cover provides a temporary covering. The removable cover may be a washable cover. Preferably, the removable cover is a disposable cover. Advantageously, the removable cover allows a quick and easy cleaning of the shield or clamp pad of the radiation protector.

In an embodiment of the radiation protector according to invention, the clamp pad has a configuration which corresponds to the configuration of the shield pad. In particular, the shield and clamp pad have a pad area with a same size . Herewith, the removable cover is attachable to either the clamp pad or the shield pad. Advantageously, just one single size of a cover can be used for both the shield and clamp pad.

The radiation protector according to the invention, the shield pad is configured to shield a thyroid from x-ray radiation. The radiation protector is a thyroid protector. The thyroid protector is arranged to be placed at a neck of a person. Preferably, the shield pad has an anatomic shape which is shaped in accordance with a shape of the neck area at a larynx. The shield pad may have a panel surface which is shaped in correspondence with a local curvature of the neck. Due to the anatomic shape of the shield pad, the covered sensitive organs do not deform in an unacceptable manner by the clamping force produced by the bracket of the radiation protector. The shield pad which is connected to the first end of the bracket can be placed against the larynx to protect sensitive organs, in particular the thyroid. The shield pad is sized to cover at least the thyroid. Preferably, the shield pad is sized to further cover a larynx. The U-shaped bracket of the thyroid protector is arranged to circumvent the neck of the person. The U-shaped bracket allows a sidewards placement of the protector. In dependence of a used x-ray device, the radiation protector may be configured in a left version, wherein the anatomic shaped shield pad is placed at a left side of a neck of a person to protect for x-ray radiation coming from the left or configured in a right version, wherein an anatomic shaped shield pad is placed at a right side of the neck of a person to protect for x-ray radiation coming from the right. In placing the shield pad at the larynx, a free second end of the bracket or a clamp pad at the second end of the bracket can be placed against a neck area at a backside of a person's body. The use of the radiation protector as a thyroid protector provides several advantages in a dental practice. Due to the presence of the U-shaped bracket, the thyroid protector can be placed quick and easy at the neck of an adult or child. In comparison with a collar shaped shield pad of a prior art protector, the shield pad of the thyroid protector according to the invention may be relatively small and configured in correspondence with the body part to be protected. The outer dimensions of the shield pad may be kept sufficiently small to allow even at a child's neck a correct and convenient placement of the thyroid protector in between a chin and sternum.

In an embodiment of the radiation protector according to the invention, the radiation protector comprises a marker to indicate a correct positioning of the radiation protector. The marker provides a reference at the radiation protector to allow an alignment of the radiation protector with respect to a body reference of a person to be imaged.
In an embodiment of the radiation protector according to the invention, the marker is positioned at the bracket. The marker may be positioned at a middle region of the bracket. Herewith, the marker may provide a reference to align the radiation protector with respect to the sagittal plane of a person.

In a preferred embodiment of the radiation protector according to the invention, the marker is positioned at the shield pad. Preferably, the marker has an oblong shape. Herewith, the marker may provide a reference to align the radiation protector with respect to a virtual line extending from an ear to a sternum of a person. Advantageously, a marker which is provided at the shield pad instead of the bracket allows a more accurate positioning, because the alignment is not affected by a possible movement of the bracket with respect to the shield pad. The oblong shape of the marker is advantageous, because this allows a correct angular alignment of the shield pad with respect to the larynx.

In an embodiment of the radiation protector according to the invention, the shield pad has an outer contour which corresponds with an outer contour of a predetermined shield area. The shield area is delimited to protect sensitive organs. Preferably, the outer contour of the shield area is delimited to protect sensitive organs of a larynx or organs situated close to the larynx, like the thyroid, extra-thoracic airway and oesophagus. Preferably, the shield pad is substantially impervious to x-ray radiation. The radiation protector according to the invention provides an advantage in that the shield pad can be placed accurately at the body part to be protected. By configuring the outer contour in correspondence with a minimal shield area, advantageously, the radiation protector leaves diagnostically interesting landmarks free for exposure to x-ray radiation. In an embodiment, the outer contour of the shield pad may be configured to protect a larynx, while still exposing a vertebral column. The vertebral column may provide a diagnostically interesting landmark in that a dentist may obtain a growth indication from a vertebra of a child.

Further, the invention may also include a disposable cover which has a bag shape and which is configured to fit around a shield pad or a clamp pad of a radiation protector according to the invention. Advantageously, the disposable cover allows a quick and easy cleaning of the radiation protector according to the invention. The disposable cover prevents a contamination of a shield pad of the protector and may quickly bring the radiation protector in a clean disinfected state before each use for each patient. By providing a possibility to quickly clean the radiation protector, a tend to omit a radiation protector during x-ray imaging will be reduced.

In an embodiment of the disposable cover according to the invention, the disposable cover is provided with an instruction, e.g. a label with a written note, to place the cover to the shield or clamp pad of the radiation protector according to the invention.

Further the invention relates to an x-ray device for taking an x-ray image of a head of a person in a sagittal plane, wherein the x-ray device comprises a radiation protector according to the invention. The x-ray device comprises an x-ray source for producing an x-ray beam in an x-ray beam direction. The x-ray device comprises an x-ray detector for receiving an incoming x-ray beam. Further, the x-ray device comprises a cephalostat for fixating a head of a person with respect to the x-ray device. The cephalostat is arranged for fixating a head of a person, such that the sagittal plane of the head is transverse to the direction of the x-ray beam. The cephalostat is situated in between the x-ray source and the x-ray detector. The cephalostat comprises a first fixating organ and a second fixating organ. The first fixating organ is situated between the second fixating organ and the x-ray source. Each of the fixating organs has a tip to be located as an opening of an external auditory canal of the head of the person. Advantageously, the use of the radiation protector according to the invention allows a natural head position of a person during a generation of a cephalogram.

Further, the invention relates to a method for taking an x-ray image of a body portion, in particular a head, of a person involving a use of a radiation protector according to the invention. In particular, the method according to the invention comprises a step of placing the radiation protector at a thyroid of a person. In particular, the method according to the invention comprises a step of replacing a disposable cover of the shield pad and/or clamp pad. In particular, the method according to the invention involves a use of an x-ray device comprising a cephalostat.

Further preferred embodiments are defined in the subclaims.

The invention will be explained in more detail with reference to the appended drawings. The drawings show a practical embodiment according to the invention, which may not be interpreted as limiting the scope of the invention. Specific features may also be considered apart from the shown embodiment and may be taken into account in a broader context as a delimiting feature, not only for the shown embodiment but as a common feature for all embodiments falling within the scope of the appended claims, in which:
Fig. 1A shows a schematic front view of a head of a person positioned in a cephalostat of an x-ray device, wherein a radiation protector according to the invention is claimed around the neck of the person;
Fig.1B and Fig. 1C show the head of the person as shown in Fig. 1A in respectively a to the front and to the back directed perspective view; and
Fig. 2 shows in a perspective view a radiation protector for protecting a thyroid against x-ray radiation according to the invention;
Fig. 3A shows a cross sectional view of a head of a person and the radiation protector as shown in Fig. 2 claimed around the neck of the person;
Fig. 3B shows a cross sectional view of a head of a person including a shielded area provided by the radiation protector according to the invention; and
Fig. 3C shows the shielded area close to a vertebral column as shown in Fig. 3B in further detail.

Identical reference signs are used in the drawings to indicate identical or functionally similar components.

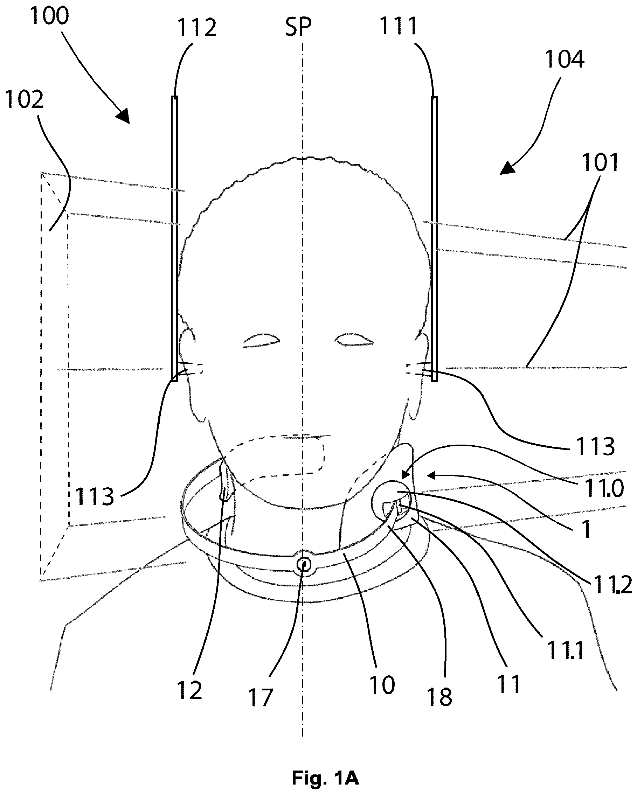

Fig. 1A-1C schematically show in several perspective views a head of a person which is positioned in an x-ray device 100 by a cephalostat 104. The x-ray device 100 is arranged for taking x-ray images of a head of a person. The cephalostat 104 is positioned in between an x-ray source 101 and an x-ray detector 102 of the x-ray device 100. The x-ray source 101 is arranged for producing an x-ray beam in an x-ray beam direction. The x-ray source 101 directs an x-ray beam to the x-ray detector 102. A produced x-ray beam originating from the x-ray source 100 is focused on an area to be radiated in a sagittal plane SP of the head of the person. The x-ray detector 102 is arranged for receiving an incoming x-ray beam. The cephalostat 104 is arranged for fixating the head of the person with respect to the x-ray source 101 and the x-ray detector 102.

The cephalostat 104 comprises a first fixating organ 111 and a second fixating organ 112. The first fixating organ 111 is positioned in between the second fixating organ and the x-ray source 101. Each fixating organ comprises a tip 113, also called an earplug, to be located at an opening of an external auditory canal of the head of the person.

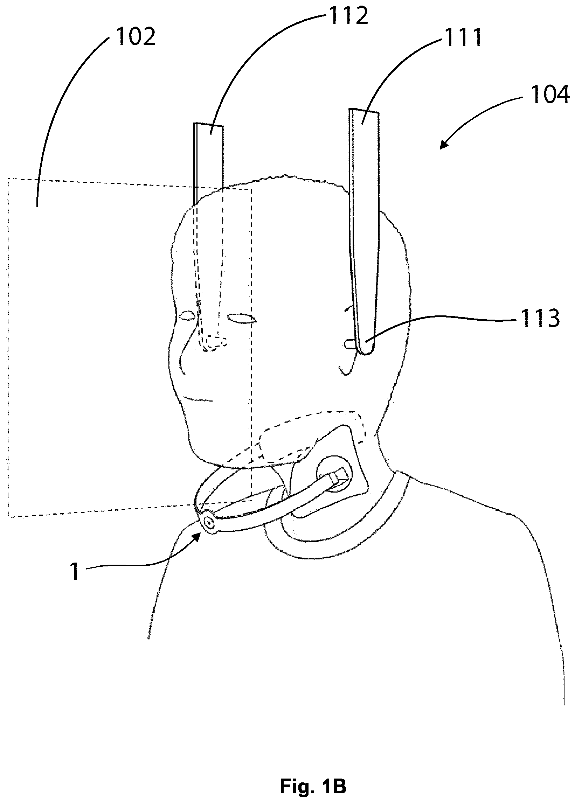

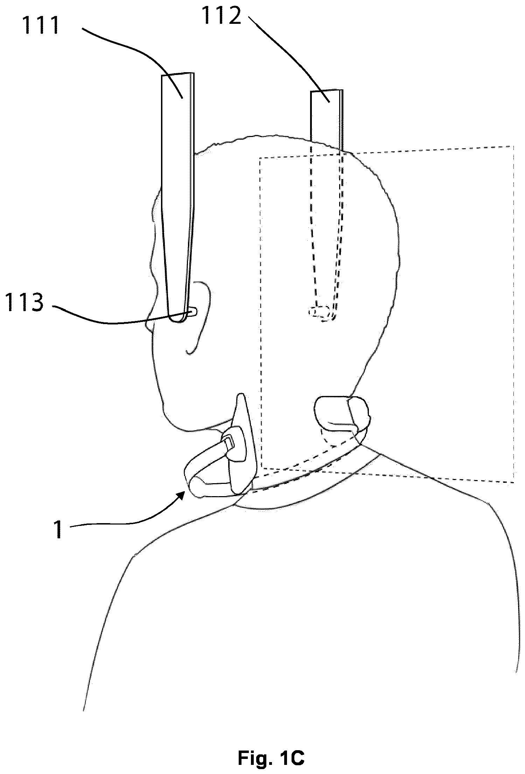

The illustrated person in figure 1 wears a radiation protector 1 according to the invention. The radiation protector 1 is placed around the neck of the person. The radiation protector 1 protects sensitive organs in the neck of the person against x-ray radiation.

The radiation protector 1 comprises a bracket 10. The bracket 10 has an U-shape. When wearing the radiation protector 1, the U-shaped bracket 10 extends at one side around the neck of the person. The neck of the person is positioned inside the U-shaped bracket 10.

The bracket 10 is resilient. The bracket 10 is bendable. The U-shape of the bracket 10 can be widened before positioning the bracket at the neck of the person. After releasing the bracket 10, the bracket 10 exerts a clamping force to maintain the radiation protector 1 in a correct position.

The bracket 10 comprises a marker 17 to bring the radiation protector in the correct position. The marker 17 may have any remarkable shape or may have a distinct colour. Here, the marker 17 includes a circular sign. The marker 17 is positioned at a middle region of the U-shaped bracket 10. Before starting the x-ray imaging, the marker 17 has to be aligned with the sagittal plane SP to bring the radiation protector in the correct position.

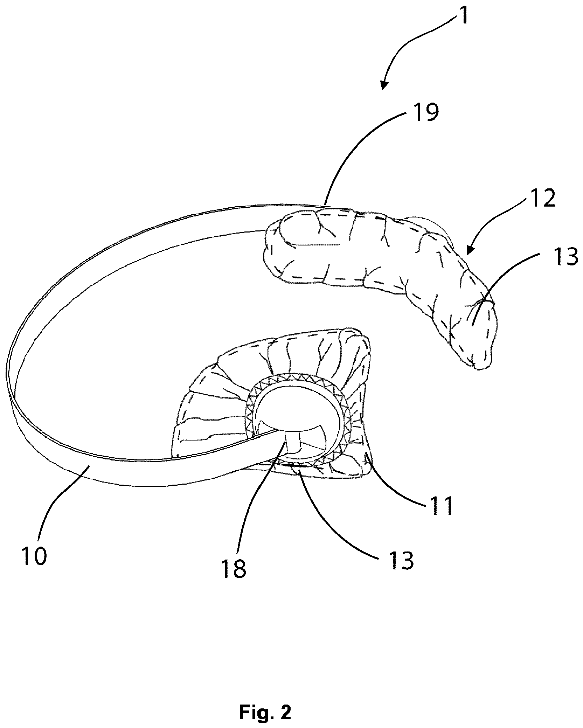

The radiation protector 1 comprises a shield pad 11 for shielding the thyroid which is a body part to be protected, see also Fig. 2. The shield pad 11 is connected to a first end 18 of the U-shaped bracket 10. When wearing the radiation protector 1, the shield pad 11 is positioned at the larynx against the skin of the person. For ergonomic reasons, the shield pad 11 has a curved panel surface has an anatomic shape. The shape is configured in correspondence with a local curvature of the neck of the person. The shield pad 11 comprises a layer of shield material for attenuating the flux of an electromagnetic radiation. Typically, the layer of shield material is a textile comprising lead particles. Preferably, the layer of shield material comprises a solid plastic layer having embedded lead particles. Preferably, the shield pad 11 comprises an impervious shield material to block x-ray radiation.

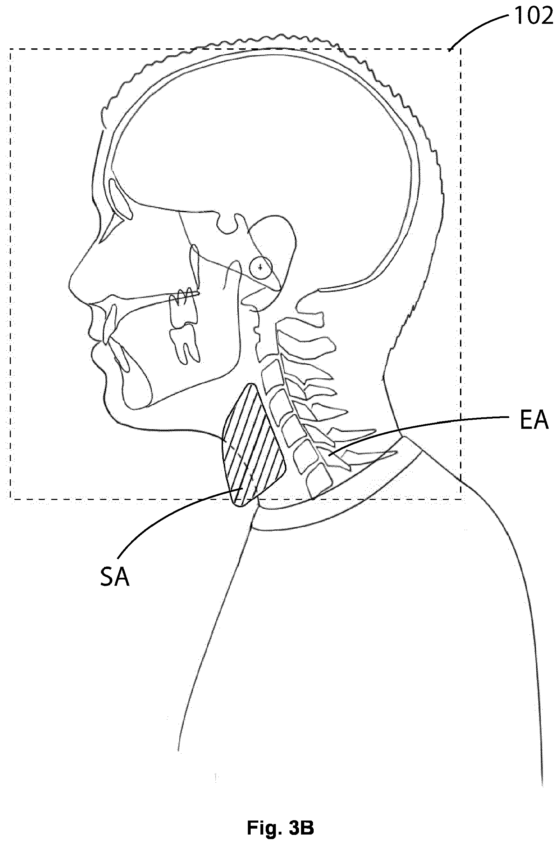

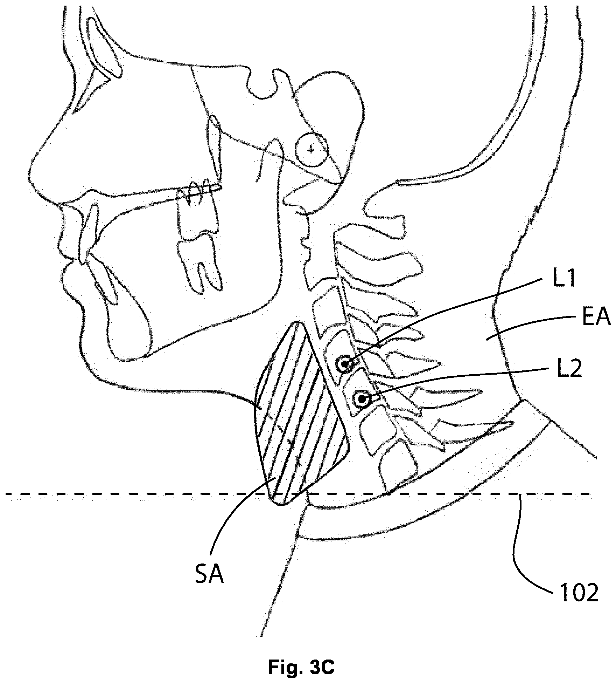

The shield pad 11 is configured to shield the thyroid of the person. The shield pad 11 has an outer contour which is in correspondence with a predetermined shield area SA, see Fig. 3B and 3C. The predetermined shield area SA is defined by a medical specialist, in particular a dentist, to protect the sensitive organs in the neck. The predetermined shield area is for example defined to cover at least the sensitive organs, like the thyroid, extra-thoracic airway and oesophagus. The outer contour of the shield pad 11 is configured to cover at least the predetermined shield area. Typically, to cover a larynx, the shield pad 11 has a panel surface which forms a pad area of at most 50 cm², in particular at most 40 cm², but preferably at most 30 cm². The outer contour of the shield pad 11 is further determined by an area to be exposed, an exposure area EA. The shield pad 11 does not cover the exposure area EA. The shield pad 11 leaves the exposure area EA free. The exposure area EA is an area of the neck of the person. The exposure area EA is determined to obtain relevant diagnostic data. The exposure area EA includes diagnostic landmarks. After imaging the diagnostic landmarks, the diagnostic landmarks provide the desired relevant diagnostic data. The diagnostic data can be used to determine for example whether a child is mature.

With respect to prior art collar shaped radiation protectors, the shown radiation protector according to the invention provides in the first place an advantage in that the radiation protector can be placed quickly from one side at the neck of a person. It is very easy to widen the bracket 10 to place the radiation protector at the neck of the person. The adapted outer contour of the shield pad 11 will stimulate a medical specialist to place the shield pad 11 at a correct position at the neck of a person. The marker 17 will assist in the correct placement. The relatively small size of the shield pad 11 will easily fit at a neck area in between a chin and a shoulder of a person. Because of the relatively small size of the shield pad 11, the radiation protector will fit in a comfortable manner to both an adult and a child. The shield pad 11 has an anatomic shape which further contributes to a wearing comfort. Additionally, it is a major advantage that the shield pad 11 leaves the exposure area EA free for x-ray imaging. A medical specialist may obtain relevant data with respect to a patient which will help the medical specialist to make a correct decision for a follow up after the diagnostics.

Fig.2 shows in a perspective view the radiation device according to the invention in further detail. Besides the above-mentioned bracket 10 and shield pad 11, the radiation device further comprises a clamp pad 12. The clamp pad 12 is connected to a second end 19 of the U-shaped bracket 10. The second end 19 of the bracket 10 is located opposite the first end. When wearing the radiation protector 1, the clamp pad 12 is positioned at the neck of the person at an area opposed the thyroid to be protected.

The clamp pad 12 is arranged to soften an engagement of the second end 19 to the neck of the person. To increase a comfort of the radiation protector and to assist in a correct placement, at least one of the shield and clamp pad 11, 12 is pivotally connected to the bracket 10. The bracket 10 is connected by a pivotal joint to respectively the shield pad 11 and/or clamp pad 12. Here, the shield pad and clamp pad 11, 12 are connected to the bracket 10 by a pivotal joint 11.0 which allows two degrees of freedom. A first degree of freedom allows a first relative rotational movement, in which the shield and clamp pad 11, 12 are pivotable with respect to the bracket 10 about a first pivot axis 11.1 substantially perpendicular to a plane defined by the bracket 10 itself. The first pivot axis 11.1 is positioned at the first end 18 of the bracket 10. A second degree of freedom allows a second relative rotational movement, in which the pad 11, 12 is rotatable with respect to the bracket 10 about a second pivot axis 11.2 extending in a direction substantially perpendicular to a plane defined by the pad itself.

The second pivot axis 11.2 is incorporated in a knob body. The second pivot axis is aligned with an axial axis of the knob body. The knob body has an inner surface which is attached to an outer surface of the pad 11, 12. The knob body is rotatable connected to the pad. The knob body has a ball shaped configuration. The knob body has an outer surface including a recess. The first end 18 of the bracket 10 is connected to the knob body at the recess. The recess has a rectangular shape. The first pivot axis 11.1 is positioned in the recess and extends in a transverse direction with respect to the recess. The first pivot axis allows the knob body to rotate with respect to the end 18 of the bracket 10. The rotatable knob body allows the bracket to rotate with respect to the pad to move the bracket away.

Advantageously, the pivotal joint 11.0 of the shield pad 11 and/or the clamp pad 12 allows the U-shaped bracket 10 to be positioned during an imaging process, such that the bracket 10 is of less disturbance. The bracket 10 can be moved away from the radiated area and may remain substantially invisible at the x-ray image.

To further increase the ergonomics, the clamp pad 12 has an anatomic shape. The clamp pad 12 has a curved panel surface 21 which forms a pad area which is configured in correspondence with a curvature of the neck of the person. Preferably, the clamp pad 12 is made of material which is penetrable for x-ray radiation, such that the clamp pad 12 does not affect an imaging. Additionally, the outer contour of the clamp pad 12 is minimally sized which further contribute to a clear imaging of the exposure area. The size of the panel surface 21 is at most 50 cm², more in particular at most 30 cm². Here, the size of the panel surface 21 is about 25 cm², which advantageously covers sensitive organs, while leaving diagnostic landmarks open for imaging.

The shield pad 11 may comprise a layered structure. The shield pad 11 comprises a layer of a shield material. The layer of shield material includes particles which attenuate a flux of electromagnetic radiation. Preferably, the particles are made of lead. The layer of shield material may comprise a textile layer, but preferably the layer comprises a plastic base which supports the particles.

The layered structure of the shield pad 11 may comprise a liner. The liner may be positioned on top of the layer of shield material. The liner may be arranged to soften a contact in between the shield pad 11 and a skin of a person. The liner may comprise a foam material. However, in a preferred embodiment, the shield pad has a rigid base layer which is anatomically formed in which no such soft liner is provided. The base is a solid base. The solid base has a smooth and closed (non-porous) outer surface at the pad area. Advantageously, such a solid base provides a high resistance against contaminations.

As further shown in Fig. 2, the radiation protector 1 further comprises a cover 13. The cover 13 is removable from the shield and clamp pad 11,12. By replacing a cover, the pad 11 may become quickly in a clean condition. A dentist may quickly replace a cover of the pad to reuse the radiation protector for a next patient. The cover may be washable, but preferably the cover is a disposable cover. Preferably, the cover 13 is made of surgical paper. The shown cover 13 is bag shaped. Advantageously, the bag shaped cover 13 allows a quick replacement. The size of the bag shaped cover is in correspondence with the size of the shield and clamp pad . The bag shaped cover has a bag opening which fits around the shield pad 11. The cover comprises a closure. Here, the closure is an elastic chord which is positioned at an edge of the bag opening.

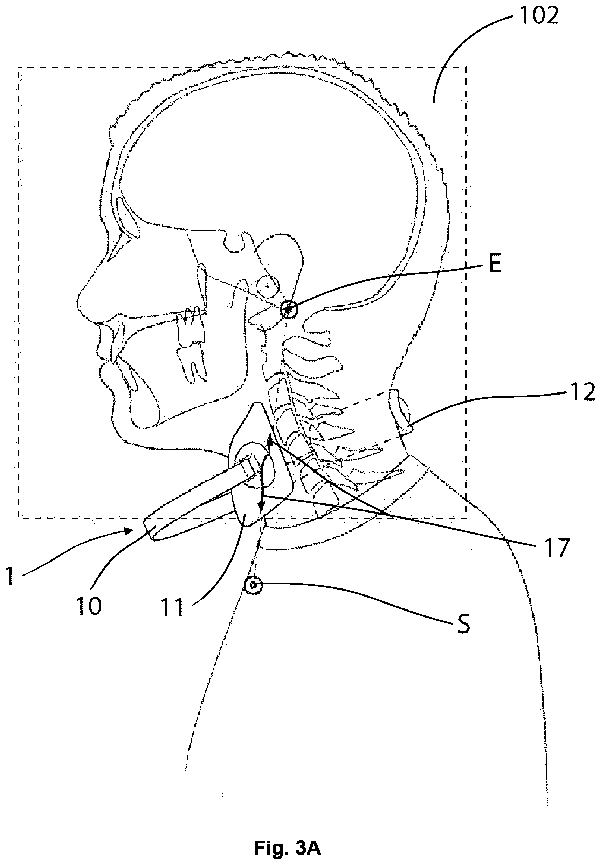

Fig. 3A-3C show in a subsequent views further details of the placement of the radiation protector 1.

Fig.3A shows in a cross-sectional side view a person who is positioned at an x-ray device 100 for imaging the head of the person. A region 102 indicated with a dashed line indicates an area of x-ray radiation. The head of the person is situated in front of the area 102. During operation of the x-ray device 100, the head of the person will be exposed to x-ray radiation. The x-ray radiation originates from an x-ray source and is extending in a slightly converging manner in a direction perpendicular to the region 102.

As illustrated in Fig. 3A, the radiation protector 1 according to the invention is placed at the neck of the person. The radiation protector 1 is placed by clamping. The radiation protector 1 is arranged as a thyroid protector to protect the thyroid from x-ray radiation. The radiation protector 1 has a bracket 10 which extends along one side, here along a right side, of the person's neck.

The radiation protector comprises a marker 17 to indicate a correct position of the radiation protector with respect to the person. Here, the marker 17 is positioned at the shield pad 11. The marker 17 is oblong and extends in an upwards direction. To obtain a correct placement of the radiation protector, the marker 17 is to be aligned with a virtual line extending from an ear E to a sternum S of a person.

Fig. 3B shows in a cross-sectional side view a shield area SA which is shielded by the shield pad 11 of the radiation protector 1. The shield area SA covers a thyroid of the person. The shield area SA has a limited surface area. The surface area of the shield area SA is limited to prevent too much shielding. Only the sensitive organs of the larynx are covered. The remaining area, the exposure area EA, of the neck of the person is exposed to x-ray radiation and visible at an x-ray image. The vertebral column is for example visible at the x-ray image.

The shield area SA and a part of the vertebral column is shown in further detail in Fig. 3C. Fig.3C shows further a first and second landmark L1, L2. The landmarks are proposed to obtain diagnostic information. The landmarks L1, L2 are positioned at a vertebra. A shape of the landmarks L1, L2 may provide a medical specialist information about a growth of a child. Advantageously, when using the radiation protector 1 according to the invention, a dentist may obtain information about the denture of the child in combination with data about its maturity.

Besides the shown embodiment of the radiation protector according to the invention, many variants are possible without leaving the scope of protection defined by the appended claims. In a variant, the clamp pad of the radiation protector may be omitted.

Although the invention has been disclosed with reference to particular embodiments, from reading this description those of skilled in the art may appreciate a change or modification that may be possible from a technical point of view but which do not depart from the scope of the invention as described above and claimed hereafter. Modifications may be made to adapt a particular situation or material to the teachings of the invention without departing from the essential scope thereof. It will be understood by those of skilled in the art that various changes may be made and equivalents may be substituted for elements thereof without departing from the scope of the invention. Therefore, it is intended that the invention is not limited to the particular embodiments disclosed in the above detailed description, but that the invention will include all embodiments falling within the scope of the appended claims.

It is further noted that the term "comprising" (and grammatical variations thereof) is used in this specification in the inclusive sense of "having" or "including", and not in the exclusive sense of "consisting only of".

Thus, the invention provides a radiation protector which can be placed quickly to a thyroid to be protected, which is convenient to a patient and which may provide further diagnostic information to a medical specialist.

## Claims

1. Thyroid radiation protector (1) for protecting a thyroid of a person from x-ray radiation comprising:
- a shield pad (11) for shielding the thyroid to be protected, in which the shield pad comprises a layer of shield material for attenuating the flux of an electromagnetic radiation;
- a fastener (10) for fastening the shield pad in abutting engagement with the thyroid to be protected, **characterised in that** the fastener comprises a resilient U-shaped bracket (10) having a first end (18) which is connected to the shield pad (11) to be placed in abutting engagement with the thyroid to be protected and a second end (19) which is situated opposite the first end (18) for engaging an area opposed the thyroid to be protected, such that in use the thyroid to be protected is situated in between the two ends (18,19) of the bracket (10) when the radiation protector (1) is clamped to the thyroid.

2. Radiation protector (1) according to claim 1, wherein the bracket (10) is pivotally connected to the shield pad (11).

3. Radiation protector (1) according to claim 1 or 2, wherein the second end (19) of the bracket is provided with a clamp pad (12) for softening an engagement of the second end (19).

4. Radiation protector (1) according to any of the claims 1-3, wherein the protector (1) comprises a bag shaped removable cover (13) which is attachable to the shield pad (11) and/or the clamp pad (12) to provide a temporary covering.

5. Radiation protector (1) according to claim 4, wherein the clamp pad (12) has a configuration which corresponds to the configuration of the shield pad (11), such that the removable cover is attachable to either the clamp pad (12) or the shield pad (11).

6. Radiation protector (1) according to claim 4, wherein the cover (13) has a bag shape which has a geometry which corresponds with the geometry of a shield pad (11) and/or clamp pad (12), such that the cover is configured to fit around a shield pad (11) and/or clamp pad (12) of the radiation protector (1).

7. Radiation protector (1) according to any of the preceding claims , wherein the bracket (10) and/or shield pad (11) comprises a marker (17) to indicate a correct position of the radiation protector (1) with respect to a body reference of the person.

8. Radiation protector (1) according to any of the preceding claims, wherein the shield pad (11) has an outer contour which corresponds with an outer contour of a predetermined shield area (SA) which is delimited to protect sensitive organs and to expose at least one diagnostic landmark (L1, L2) situated close to the shield area (SA).

9. Radiation protector (1) according to any of the preceding claims, wherein the disposable cover (13) comprises an instruction for placing the cover to the shield pad (11) of the radiation protector.

10. X-ray device (100) for taking an x-ray image of a head of a person in a sagittal plane comprising:
- an x-ray source (101) for producing an x-ray beam in an x-ray beam direction;
- an x-ray detector (102) for receiving an incoming x-ray beam; and
- a cephalostat (104) for fixating the head of the person, such that the sagittal plane of the head is transverse to the direction of the x-ray beam, said cephalostat (104) being situated between the x-ray source (101) and the x-ray detector (102), in which said cephalostat (104) comprises a first fixating organ (111) and a second fixating organ (112) each having a tip (113) to be located at an opening of an external auditory canal of the head of the person for fixating the head with respect to the x-ray device (100), in which the first fixating organ (111) is situated between the second fixating organ (112) and the x-ray source (101), wherein the x-ray device (100) comprises a radiation protector (1) according to any of the claims 1-9.

11. Method for taking an x-ray image of a portion of a head of a person involving the use of a radiation protector (1) according to any of the claims 1-9, wherein the method comprising a step of:
- placing the radiation protector (1) at a thyroid of a person.

12. Method according to claim 11, wherein the method comprises a step of aligning a marker (17) of the radiation protector (1) with respect to a body reference, in particular a sagittal plane (SP) or a virtual line extending from an ear (E) to a sternum (S) of a person.

13. Method according to claim 11 or 12, comprising the step of:
- replacing a disposable cover (13) of the shield pad and/or clamp pad.

14. Method according to any of the claims 11-13, involving the use of an x-ray device (100) according to claim 10.

## Patentansprüche

1. Schilddrüsenstrahlungsschutz (1) zum Schutz einer Schilddrüse einer Person vor Röntgenstrahlung mit:
- einem Abschirmkissen (11) zur Abschirmung der zu schützenden Schilddrüse, wobei das Abschirmkissen eine Schicht aus Abschirmmaterial zur Dämpfung des Flusses einer elektromagnetischen Strahlung aufweist;
- einem Befestigungselement (10) zur Befestigung des Abschirmkissens in enger Anlage an der zu schützenden Schilddrüse, **dadurch gekennzeichnet, dass** das Befestigungselement einen federnden U-förmigen Bügel (10) umfasst, der ein erstes Ende (18), das mit dem Abschirmkissen (11) verbunden ist, welches in enge Anlage an der zu schützenden Schilddrüse anzuordnen ist, und ein dem ersten Ende (18) gegenüberliegendes zweites Ende (19) hat, das in Anlage an einem der zu schützenden Schilddrüse gegenüberliegenden Bereich angeordnet wird, so dass sich im Gebrauch die zu schützende Schilddrüse zwischen den beiden Enden (18,19) des Bügels (10) befindet, wenn der Strahlungsschutz (1) an der Schilddrüse angebracht ist.

2. Strahlungsschutz (1) nach Anspruch 1, wobei der Bügel (10) schwenkbar mit dem Abschirmkissen (11) verbunden ist.

3. Strahlungsschutz (1) nach Anspruch 1 oder 2, wobei das zweite Ende (19) des Bügels mit einem Klemmpolster (12) versehen ist, um eine weichere Anlage des zweiten Endes (19) zu erreichen.

4. Strahlungsschutz (1) nach einem der Ansprüche 1 - 3, wobei der Strahlungsschutz (1) eine beutelförmige entfernbare Abdeckung (13) umfasst, die an dem Abschirmkissen (11) und/oder dem Klemmpolster (12) befestigbar ist, um eine temporäre Abdeckung bereitzustellen.

5. Strahlungsschutz (1) nach Anspruch 4, wobei das Klemmpolster (12) eine Konfiguration aufweist, die der Konfiguration des Abschirmkissens (11) entspricht, so dass die entfernbare Abdeckung entweder an dem Klemmpolster (12) oder dem Abschirmkissen (11) befestigbar ist.

6. Strahlungsschutz (1) nach Anspruch 4, wobei die Abdeckung (13) die Form eines Beutels aufweist, der eine Geometrie hat, welche der Geometrie eines Abschirmkissens (11) und/oder Klemmpolsters (12) entspricht, derart, dass die Abdeckung so ausgebildet ist, dass sie um ein Abschirmkissen (11) und/oder Klemmpolster (12) des Strahlungsschutzes (1) passt.

7. Strahlungsschutz (1) nach einem der vorhergehenden Ansprüche, wobei der Bügel (10) und/oder das Abschirmkissen (11) einen Marker (17) umfasst, um eine korrekte Position des Strahlungsschutzes (1) bezüglich einer Körperreferenz der Person anzuzeigen.

8. Strahlungsschutz (1) nach einem der vorhergehenden Ansprüche, wobei das Abschirmkissen (11) eine Außenkontur aufweist, die einer Außenkontur eines vorbestimmten Abschirmbereichs (SA) entspricht, die begrenzt ist für einen Schutz von empfindlichen Organen und für ein Freilassen mindestens einer nahe dem Abschirmbereich (SA) liegenden diagnostischen Markierung (L1, L2).

9. Strahlungsschutz (1) nach einem der vorhergehenden Ansprüche, wobei die Einweg-Abdeckung (13) eine Anweisung zum Aufsetzen der Abdeckung auf das Abschirmkissen (11) des Strahlungsschutzes umfasst.

10. Röntgeneinrichtung (100) zur Aufnahme eines Röntgenbildes eines Kopfes einer Person in einer Sagittalebene mit:
- einer Röntgenquelle (101) zur Erzeugung eines Röntgenstrahls in einer Röntgenstrahlrichtung ;
- einem Röntgendetektor (102) zur Aufnahme eines einfallenden Röntgenstrahls; und
- einem Cephalostat (104) zur Fixierung des Kopfes der Person, so dass die Sagittalebene des Kopfes quer zur Richtung des Röntgenstrahls verläuft, wobei der Cephalostat (104) zwischen der Röntgenquelle (101) und dem Röntgendetektor (102) angeordnet ist, wobei der Cephalostat (104) ein erstes Fixierorgan (111) und ein zweites Fixierorgan (112) umfasst, die jeweils eine Spitze (113) haben, die an einer Öffnung eines äußeren Gehörgangs des Kopfes der Person zur Fixierung des Kopfes gegenüber dem Röntgengerät (100) angeordnet wird, wobei das erste Fixierorgan (111) sich zwischen dem zweiten Fixierorgan (112) und der Röntgenquelle (101) befindet, wobei das Röntgengerät (100) einen Strahlungsschutz (1) nach einem beliebigen der Ansprüche 1 -9 umfasst.

11. Verfahren zur Aufnahme eines Röntgenbildes eines Teils eines Kopfes einer Person unter Verwendung eines Strahlungsschutzes (1) nach einem beliebigen der Ansprüche 1 - 9, wobei das Verfahren den folgenden Schritt umfasst:
- Platzieren des Strahlungsschutzes (1) an einer Schilddrüse einer Person.

12. Verfahren nach Anspruch 11, wobei das Verfahren einen Schritt des Ausrichtens eines Markers (17) des Strahlungsschutzes (1) bezüglich einer Körperreferenz, insbesondere einer Sagittalebene (SP) oder einer virtuellen Linie, die sich von einem Ohr (E) zu einem Brustbein (S) einer Person erstreckt, umfasst.

13. Verfahren nach Anspruch 11 oder 12, umfassend den folgenden Schritt:
- Ersetzen einer Einweg-Abdeckung (13) des Abschirmkissens und/oder des Klemmpolsters.

14. Verfahren nach einem der Ansprüche 11 - 13, wobei ein Röntgengerät (100) nach Anspruch 10 verwendet wird.

## Revendications

1. Protecteur (1) contre les radiations pour protéger une thyroïde d'une personne contre une exposition à des rayons X comprenant:
- une plaque de blindage (11) pour protéger la thyroïde à protéger, dans lequel la plaque de blindage comprend une couche de matériau protecteur pour atténuer le flux d'un rayonnement électromagnétique ;
- un élément de fixation (10) pour attacher la plaque de blindage en butée contre la thyroïde à protéger, **caractérisée en ce que** l'élément de fixation comprend un support élastique en forme de U (10) ayant une première extrémité (18) qui est reliée à la plaque de blindage (11) à placer en butée contre la thyroïde à protéger et une seconde extrémité (19) qui est située en regard de la première extrémité (18) pour engager une zone opposée à la thyroïde à protéger, de telle sorte qu'en cours d'utilisation la thyroïde à protéger est située entre les deux extrémités (18, 19) du support (10) lorsque le protecteur (1) contre les radiations est fixé à la thyroïde.

2. Le protecteur (1) contre les radiations selon la revendication 1, dans lequel le support (10) est relié de manière pivotante à la plaque de blindage (11).

3. Le protecteur (1) contre les radiations selon la revendication 1 ou 2, dans lequel la seconde extrémité (19) du support est munie d'un patin de serrage (12) pour adoucir un engagement de la seconde extrémité (19).

4. Le protecteur (1) contre les radiations selon l'une quelconque des revendications 1 à 3, dans lequel le protecteur (1) comprend une housse (13) amovible en forme de sac qui peut être fixée à la plaque de blindage (11) et/ou au patin de serrage (12) pour fournir une enveloppe temporaire.

5. Le protecteur (1) contre les radiations selon la revendication 4, dans lequel le patin de serrage (12) a une configuration qui correspond à la configuration de la plaque de blindage (11), de sorte que la housse amovible peut être attachée soit au patin de serrage (12) soit à la plaque de blindage (11).

6. Le protecteur (1) contre les radiations selon la revendication 4, dans lequel la housse (13) a une forme de sac qui a une géométrie qui correspond à la géométrie d'une plaque de blindage (11) et/ou d'un patin de serrage (12), de sorte que la housse est configurée pour s'adapter autour d'une plaque de blindage (11) et/ou d'un patin de serrage (12) du protecteur (1) contre les radiations.

7. Le protecteur (1) contre les radiations selon l'une quelconque des revendications précédentes, dans lequel le support (10) et/ou la plaque de blindage (11) comprend un repère (17) pour indiquer une position correcte du protecteur (1) contre les radiations par rapport à une référence corporelle de la personne.

8. Le protecteur (1) contre les radiations selon l'une quelconque des revendications précédentes, dans lequel la plaque de blindage (11) a un contour extérieur qui correspond à un contour extérieur d'une zone de protection prédéterminée (SA) qui est délimitée pour protéger des organes sensibles et pour exposer au moins un repère de diagnostic (L1, L2) situé à proximité de la zone de protection (SA).

9. Le protecteur (1) contre les radiations selon l'une quelconque des revendications précédentes, dans lequel la housse (13) jetable comprend une instruction pour placer la housse sur la plaque de blindage (11) du protecteur contre les radiations.

10. Dispositif à rayons X (100) pour prendre une image radiographique d'une tête d'une personne dans un plan sagittal comprenant:
- une source (101) de rayons X pour produire un faisceau de rayons X dans une direction de faisceau de rayons X ;
- un détecteur (102) de rayons X pour recevoir un faisceau de rayons X entrant ; et
- un céphalostat (104) pour fixer la tête de la personne, de manière telle que le plan sagittal de la tête est transversal à la direction du faisceau de rayons X, ledit céphalostat (104) étant situé entre la source (101) de rayons X et le détecteur (102) de rayons X, dans lequel ledit céphalostat (104) comprend un premier élément de fixation (111) et un second élément de fixation (112) ayant chacun une pointe (113) devant être située à une ouverture d'un canal auditif externe de la tête de la personne pour fixer la tête par rapport au dispositif à rayons X (100), dans lequel le premier élément de fixation (111) est situé entre le second élément de fixation (112) et la source (101) de rayons X, dans lequel le dispositif à rayons X (100) comprend un protecteur (1) contre les radiations selon l'une quelconque des revendications 1 à 9.

11. Procédé pour prendre une image radiographique d'une partie d'une tête d'une personne impliquant l'utilisation du protecteur (1) contre les radiations selon l'une quelconque des revendications 1 à 9, dans lequel le procédé comprend une étape consistant à:
- placer le protecteur (1) contre les radiations au niveau d'une thyroïde de la personne.

12. Le procédé selon la revendication 11, dans lequel le procédé comprend une étape d'alignement d'un marqueur (17) du protecteur (1) contre les radiations par rapport à une référence du corps, en particulier un plan sagittal (SP) ou une ligne virtuelle s'étendant d'une oreille (E) au sternum (S) d'une personne.

13. Le procédé selon la revendication 11 ou 12, comprenant l'étape consistant à:
- remplacer la housse (13) jetable de la plaque de blindage et/ou du tampon de serrage.

14. Le procédé selon l'une quelconque des revendications 11 à 13, impliquant l'utilisation d'un dispositif à rayons X (100) selon la revendication 10.
